# EUROPÄISCHE PATENTSCHRIFT

(11) **EP 2 395 944 B1**
(45) Veröffentlichungstag und Bekanntmachung des Hinweises auf die Patenterteilung: **23.09.2020**
(21) Anmeldenummer: 09775838.7
(22) Anmeldetag: 11.02.2009
(51) Int. Cl.: A61F 2/24

(54) **Kathetersystem zur Rekonstruktion einer anatomischen Struktur**
Cathetersystem for reconstruction of an anatomic structure
Système de cathéter pour la reconstruction d'une structure anatomique

(43) Veröffentlichungstag der Anmeldung: 21.12.2011
(73) Patentinhaber: Lutter, Georg, 24105 Kiel (DE); Lozonschi, Lucian, Madison, WI 53717 (US)
(72) Erfinder: Lutter, Georg, 24105 Kiel (DE); Lozonschi, Lucian, Madison, WI 53717 (US)
(74) Vertreter: Westphal, Mussgnug & Partner Patentanwälte mbB
(86) Internationale Anmeldenummer: PCT/DE2009/000176
(87) Internationale Veröffentlichungsnummer: WO 2010/091653

(56) Entgegenhaltungen:
- WO-A-02/28321
- DE-A1-102007 043 831
- US-A1- 2006 025 784
- US-A1- 2007 233 239

## Beschreibung

Die Erfindung betrifft einen Kathetersystem bestehend aus einen Katheter, mit einem flexiblen, entlang seiner Längserstreckung wenigstens zwei Hohlräume ausbildenden Katheterkörper. Die Erfindung betrifft insbesondere einen Katheter zur Rekonstruktion einer Herzklappe, insbesondere der Mitralklappe.

Katheter werden in einer Vielzahl von unterschiedlichen Ausbildungen zur minimal-invasiven Diagnostik und Therapie eingesetzt. Gewöhnlich weisen Katheter einen oder mehrere Hohlräume auf, in denen Flüssigkeiten zu- oder abgeleitet, oder chirurgisches Werkzeug und Transplantate geführt werden können.

Insbesondere bei der Herzklappenablation werden Katheter eingesetzt, die neben flüssigkeitsleitenden Hohlräumen gleichzeitig Hohlräume zur Aufnahme von Werkzeugen und zum Einbringen eines Herzklappenstents zum Ersatz einer fehlgebildeten oder krankhaft veränderten Herzklappe aufweisen.

Beispielsweise wird bei Vorliegen von Erkrankungen der Mitralklappe versucht, die alte Herzklappe trotz aufgetretener Fehlfunktion weitestgehend zu erhalten, damit der gesamte dynamische Mitralklappenapparat nicht zerstört wird. Die Sebnenfäden, die an der Mitralklappe ansetzen, sind z.B. sehr wichtig für die Ventrikelfunktion und sollten daher möglichst nicht von der alten Mitralklappe losgelöst werden. Um bei Hochrisikopatienten die Herz-Lungen-Maschine und einen Eingriff am "offenen" Herzen zu vermeiden, wurden minimal-invasive, z.B. transapikale, oder perkutane Methoden zur Implantation einer Herzklappe mit Hilfe von verschiedenen Kathetersystemen entwickelt.

So ist aus der DE 195 46 692 C2 und der EP 1 469 797 B1 eine selbstexpandierbare Herzklappenprothese zur Implantation im menschlichen Körper über ein Kathetersystem mit einer Herzklappe und mit einem mit der Herzklappe verbundenen zusammenfaltbaren und expandierbaren Stent bekannt. Eine solche selbstexpandierbare Herzklappenprothese kann mit Hilfe eines Kathetersystems durch eine Leistenarterie bis hin zum Implantationsort am Herzen geführt werden und sukzessiv entfaltet werden. Nach der Entfaltung kann die Herzklappenprothese auch mit Unterstützung von Verankerungshaken zumindest im herznahen Bereich im jeweiligen Blutgefäss verankert werden. Die eigentliche Herzklappenprothese befindet sich dabei im Stent.

Eine weitere Vorrichtung zur Befestigung und Verankerung von Herklappenprothesen ist in der DE 100 10 074 A1 beschrieben, die im Wesentlichen aus drahtförmigen miteinander verbundenen Elementen gebildet wird. Eine verbesserte Positionierung und angulare Ausrichtung zur Aortenklappe kann durch den Stent der EP 1 469 797 B1 erreicht werden, bei dem sogenannte Stützbügel ausgebildet sind, die in die Aortentaschen einführbar sind und so eine definierte Entfernung zur Aortenklappe herstellen.

Darüber hinaus besteht auch die Möglichkeit eine fehlgeschlagene Implantation einer Herzklappenprothese abzubrechen und den Klappenstent (d.h. die in einen Stent integrierte Klappe) wieder in das Kathetersystem (genauer die Kartusche) hineinzuziehen. Hierbei wird dann der gesamte Stent wieder zusammengefaltet und in die Katheteröffnung (Kartusche) zurückgeführt, um ihn dann nach besserer Positionierung wieder an anderer Position auszufahren.

Ein weitaus größeres Problem für die optimale Platzierung der neuen Herzklappe im Stent (bzw. Klappenstent) besteht jedoch darin, dass die alte, native Klappe in den meisten Fällen der zuvor beschriebenen Implantationstechnik nicht entfernt werden soll. Allerdings führt dieses dazu, dass die neue Klappe, die in eine alte, deformierte Klappe hineingedrückt (teilweise hineingequetscht) wird, in ihrer ursprünglichen Stentform abgewandelt wird. Dies liegt daran, dass der Implantationsort für den Klappenstent durch die Morphologie, den Zustand und die Beschaffenheit der alten nativen Klappe bestimmt wird (z.B. bei Klappensklerose oder -verkalkung der nativen Klappe).

Weiterhin sind auch Perfusionskatheter oder aufblasbare Einheiten beschrieben, um in kleineren oder größeren (aortalen) Gefäßen eine isolierte Kammer zur Klappenresektion herstellen zu können:
Bei der Herzoperation kommen spezielle Perfusionskatheter, die vom Prinzip der Ballonkatheter (DE 195 33 601) bekannt sind, zum Einsatz. Die US 6,135,981 schlägt beispielsweise einen Perfusionskatheter mit zwei distal benachbart angeordneten, aufblasbaren Kammern vor, die einen von den Kammern eingeschlossenen Operationsraum bilden, der vom Blutkreislauf ausgenommen ist. Zur verbesserten Positionierung des Ballons im Gefäß kann die Oberfläche speziell (Noppen, Ausbuchtungen) verändert sein (US 5,423,745). Für die Ablation von erkrankten Herzklappen ist die Vorrichtung mit zwei entlang des Katheters angeordneten, inflatierbaren Dilatationseinheiten bekannt (siehe DE 102 17 559).

Besonders wünschenswert wäre es allerdings zumindest im Fall einer Herzinsuffizienz, statt einer vollständigen oder bevorzugt teilweisen Entfernung einer fehlgebildeten oder krankhaft veränderten Herzklappe, den Klappenannulus der defekten Herzklappe des Patienten mit den dazugehörigen alten Klappenanteilen mit einfachen Mitteln so zu verändern und zu rekonstruieren, dass die Insuffizienz der Klappe beseitigt werden kann.

Als weiterer Stand der Technik werden die US 2006/025784 A1, US 2007/233239 A1 und WO 02/28321 A genannt.

Aufgabe der Erfindung ist es daher, einen Katheter zu schaffen, der mit einfachen Mitteln eine Rekonstruktion von Gefäßquerschnitten, insbesondere die Rekonstruktion einer insuffizienten Herzklappe ermöglicht.

Die Aufgabe wird erfindungsgemäß durch den Katheter mit den Merkmalen von Anspruch 1 gelöst. Die Unteransprüche geben vorteilhafte Ausgestaltungen der Erfindung wieder.

Der Erfindung liegt der Gedanke zugrunde, einen Katheter abschnittsweise derart auszubilden, dass sich der flexible Katheter im Wesentlichen ringförmig um eine zu rekonstruierende Struktur, beispielsweise eine Herzklappe, legen und an dieser dauerhaft fixiert werden kann, wobei die einzelnen Abschnitte des Katheters in ihrer Länge veränderbar sind, sodass die Anatomie des Gefäßes/der Klappe verändert und damit eine Änderung des Querschnitts des betroffenen Gefäßes erreicht und z.B. so eine Herzklappeninsuffizienz beseitigt werden kann.

Entscheidend ist also, dass durch den erfindungsgemäßen Katheter der native Annulus an den richtigen Stellen/in den richtigen Abschnitten des ringförmig ausgebildeten Katheters zu sammengezogen werden kann. Ebenso wichtig ist dabei auch die optimale Fixierung des Kathers am Gewebe.

Hierzu weist der erfindungsgemäße Katheter zur Klappenrekonstruktion besondere Fixationselemente auf, die den Katheter, ähnlich der in der PCT/DE2005/000437 vorgeschlagenen Ausbildung, an das Gefäßgewebe eng und fluiddicht fixieren. Auch kann dabei auf die WO 00/74574 A1 zurückgegriffen werden, die einen Gegenstand zeigt, der sich ringförmig an Gewebe ansaugen kann. Jedoch geht aus diesen Druckschriften nicht hervor, wie durch diese Fixierung eine dauerhafte Veränderung eines Gefäßquerschnitts bewirkt werden könnte.

Um eine dauerhafte und stabile Rekonstruktion des Gefäßquerschnitts zu ermöglichen, die auch mit minimal-invasiven Techniken durchführbar sein sollte, bildet der erfindungsgemäße Katheter einen faltbaren, repositionierbaren, zu einem Klappenring ausbildbaren Katheter mit besonders flexibler Form und Struktur, der mittels Strukturen zum Ansaugen an den Herzklappenring fixiert werden kann. Der Katheter wird perkutan oder transapikal mittels eines Hüllkatheters oder durch ein dem Herzen angrenzendes, großes Gefäß in eine Herzkammer eingeführt und dort entfaltet. Seine Fixationselemente werden so justiert, dass sich zum einen eine optimale Fixation ergibt und zum anderen durch in ihrer Länge unterschiedlich auszubildende Abschnitte im bevorzugt ringförmig angeordneten Katheter auch die native Annuluskonfiguration rekonstruiert und optimiert werden kann.

Die Erfindung wird anhand von in den Zeichnungen dargestellter bevorzugter Ausführungsbeispiele näher erläutert. Es zeigen:
- Fig. 1: einen Transversalschnitt durch ein menschliches Herz mit Draufsicht auf die Mitralklappe mit dem Katheter nach der Erfindung *in situ;*
- Fig. 2: eine Lateralansicht des erfindungsgemäßen Katheters zur Klappenrekonstruktion; und
- Fig. 3: ein als Klappenstent ausgebildetes Ausführungsbeispiel der vorliegenden Erfindung.

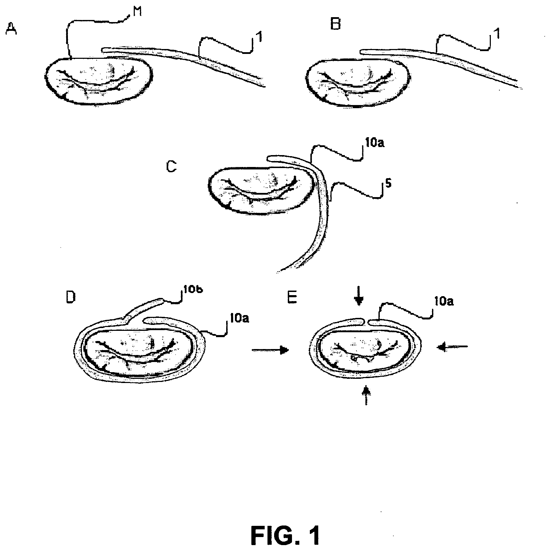

Fig. 1 zeigt zur beispielhaften Erläuterung der Erfindung einen Transversalschnitt durch ein menschliches Herz mit Draufsicht auf die Mitralklappe (M) mit dem erfindungsgemäßen Katheter 1. In der äußeren Ansicht ähnelt der erfindungsgemäße Katheter 1 einem herkömmlichen flexiblen Katheter, der gemäß einer bevorzugten Ausgestaltung durch (nicht gezeigte) Bowdenzüge in eine Ringkonformation überführt werden kann und sich an den Annulus einer erkrankten, insuffizienten Mitralklappe M anschmiegen kann. Nach genauer Positionierung und erfolgter Fixierung des Katheters 1 an der Mitralklappe M können die einzelnen Abschnitte des Katheters in ihrer Länge so verkürzt oder verlängert werden, dass die Mitralklappe M wieder suffizient wird (Fig. 1E).

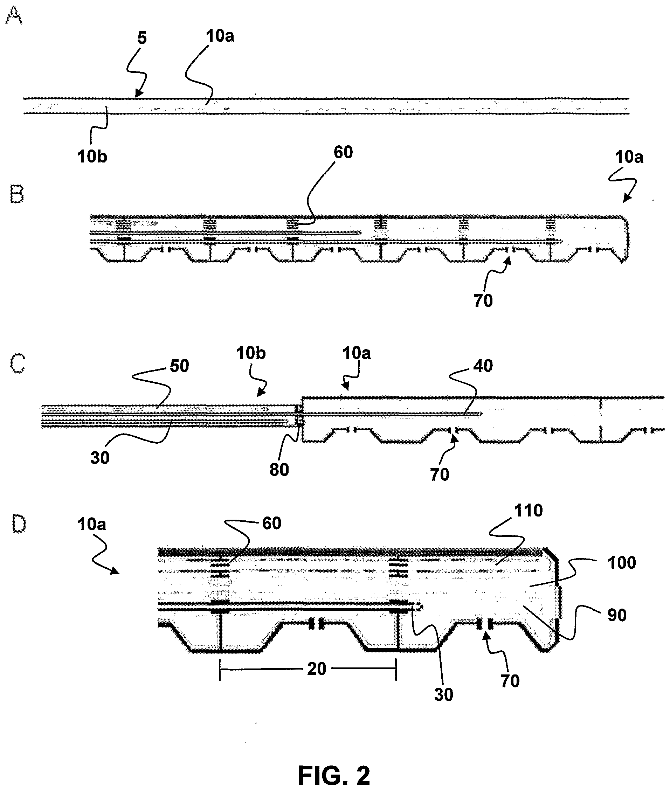

Fig. 2 zeigt einen Längsschnitt eines besonders bevorzugt ausgebildeten Katheters nach der Erfindung in vier Detailansichten:

Fig. 2A zeigt ein besonders bevorzugt ausgebildetes Kathetersystem 10, bestehend aus dem erfindungsgemäßen Katheter 10a, einem lösbar mit dem Katheter 10a verbundenen Katheterträger 10b und einer den Katheter 10a und den Katheterträger 10b aufnehmenden Hüllkatheter 5. Der Hüllkatheter ist bevorzugt im Bereich seines freien Endes beispielsweise durch Bowdenzüge auslenkbar eingerichtet, sodass der Katheter 10a in einer vorgegebenen Richtung aus dem Hüllkatheter 5 vorgeschoben und der in Fig.1 gezeigten Mitralklappe M angepasst werden kann.

Der Katheter 10a und der Katheterträger 10b weisen bevorzugt eine Kopplungseinrichtung 80 zum lösbaren Verbinden des Katheters 10a mit dem Katheterträger 10b auf (Fig. 2C), wobei die Kopplungseinrichtung 80 während des Entkopplungsvorgangs des Katheterträgers 10b vom Katheter 10a zum fluiddichten Verschließen der im Katheter 10a geführten Hohlräume eingerichtet ist. Die Kopplungsvorrichtung ist notwendig, um den Katheter 10a bei minimalinvasiver Implantationstechnik den Katheter 10a dauerhaft in den Patienten zu verbringen.

In Fig. 2B ist nun der Katheter 10a in einer größeren Ansicht dargestellt, wobei zu erkennen ist, dass der Katheter 10a durch Schleusen 60 in Abschnitte unterteilt ist. Fig. 2D verdeutlicht dieses weiter:

Jeder durch eine Schleuse 60 gebildete Abschnitt 20 weist im gezeigten Ausführungsbeispiel drei sich in Längsrichtung des Abschnitts 20 erstreckende Hohlräume 90, 100, 110 auf, wobei die im Beispiel dargestellten oberen zwei Hohlräume 100, 110 auch zu einem einzigen Hohlraum 100/110 zusammengefasst sein können. In den Hohlräumen 90, 100, 110 sind jeweils Schleusen 60 angeordnet, die die Hohlräume 90, 100, 110 abschnittsweise unterteilen. Die Schleusen 60 dichten die Hohlräume 90, 100, 110 jedes Abschnitts 20 gegeneinander ab, können aber beispielsweise durch Beauschlagung eines vorbestimmten Drucks geöffnet werden, sodass die Hohlräume 90, 100, 110 jedes Abschnitts 20 miteinander kommunizieren.

Bevorzugt sind die Schleusen jedoch nur durch besondere im Katheterträger 10b vorgehaltene Elemente 30, 40, 50 (vgl. Fig. 2C) zu öffnen, die durch die Hohlräume 90, 100, 110 in ihrer Längsrichtung durch die Abschnitte 20 vorgeschoben werden können.

Das Element 30 dient dabei bevorzugt dem Anlegen eines Unterdrucks an die wenigstens eine Öffnung 70 eines Abschnitts zur Fixation des Katheters 10a an dem Gewebe. Da es schwierig sein mag eine dauerhafte Fixation durch Unterdruck zu erreichen, kann alternativ auch vorgesehen sein, dass das Element 30 über die Öffnungen 70 ein Klebemittel (beispielsweise einen Fibrinkleber) abgibt und derart für eine Fixierung am Gewebe sorgt.

Die Elemente 40, 50 dienen zum Bewirken einer Längenänderung eines einzelnen Abschnitts 20. Die Längenänderung eines einzelnen Abschnitts 20 kann beispielsweise durch Einleiten oder Absaugen eines Fluids (Gas oder Flüssigkeit) hervorgerufen werden. Wird beispielsweise ein Abschnitt 20 mit einem Überdruck eines Fluids beaufschlagt, wird sich der Abschnitt 20 des flexibel ausgebildeten Katheters 10a dehnen, sodass der durch wenigstens eine Schleuse 60 abgedichtete Abschnitt 20 verlängern wird. Umgekehrt kann eine Verkürzung eines Abschnitts 20 bewirkt werden, wenn ein Unterdruck in einem Abschnitt 20 erzeugt wird, sodass sich der Abstand zwischen zwei Schleusen verringert. Nach der beispielhaft gezeigten Ausbildung ist vorgesehen, dass die Elemente 40, 50 nach erfolgter Fixation am Gewebe bis in den vordersten Abschnitt 20 vorgeschoben werden, die Längenänderungen des vordersten Abschnitts 20 bewirkt wird und die hinter dem vordersten Abschnitt 20 liegenden Abschnitte 20 sukzessive verändert werden. Dabei sind die Schleusen 60 die Abschnitte 20 gegeneinander derart abdichtend ausgebildet, dass nahezu keine Volumenänderungen auftreten, wenn ein Element 40, 50 den Abschnitt durchfahren und wieder verlassen hat.

Zusätzlich kann im Katheter 10a ein Bowdenzug vorgesehen sein, der nicht nur zur Auslenkung des Katheters 10a verwendet, sondern zur abschnittsweisen Verkürzung des jeweiligen Abschnitts 20 eingerichtet sein kann.

Durch die jeweilige Längenänderungen der Abschnitte 20, die eine Querschnittsänderung des ringförmig angelegten Katheters 10a bewirken, kann bei gleichzeitiger Fixation mittels der Öffnungen 70 in den jeweiligen Abschnitten 20 am Gewebe ein bestimmter Abschnitt des Klappenrings verkürzt, aber auch geweitet werden, um so z.B. die vorhandene Mitralklappeninsuffizienz aufzuheben.

Eine weitere Möglichkeit besteht darin (nicht dargestellt), dass es nur ein Element 30, 40, 50 gibt, das alle zwei oder mehr Hohlräume 90, 100, 110 der jeweiligen Untereinheit 20 bedienen kann, so dass nicht unbedingt drei einzelne Elemente 30, 40, 50 vorhanden sein müssen. Diese kann beispielsweise derart bewerkstelligt werden, dass eine besondere Führung dieses einen Elements in der Kopplung 80 erfolgt, so dass dieses durch besondere Markierungen (für den behandelnden Arzt wichtig) in den jeweiligen, Hohlraum 90, 100, 110 eingeführt werden kann.

Sollte es, wie oben bereits bemerkt, technisch schwierig sein, allein durch die Poren 70 der Abschnitte 20 ein Vakuum herzustellen, um den Katheter 10a am Klappenannulus zu fixieren, wird eine Injektion von einem Klebemittel (z.B. Kleber, Fibrinkleber) in den jeweiligen Hohlraum notwendig sein, so dass sich der Volumenzustand des Hohlraums 90, 100, 110 im jeweiligen Abschnitt 20 nicht mehr ändert, bzw. nur unter kontrollierten Bedingungen verändert werden kann. So kann es mit Hilfe von Polymerschäumen, insbesondere solchen, die während der Polymerisierung Gase freisetzen (z.B. Polyurethanschäume), möglich sein, einen dauerhaften Über- oder Unterdruck aufzubauen. Die entsprechenden Monomere oder Hilfsstoffe (z.B. Wasser zur Schaumbildung) müssten dann in z.B. flüssiger oder Gas-Form exakt zudosiert werden und würden im Zuge ihrer Erhärtung aufschäumen und das entsprechende Volumen (durch Volumenzu- oder Abnahme) einnehmen.

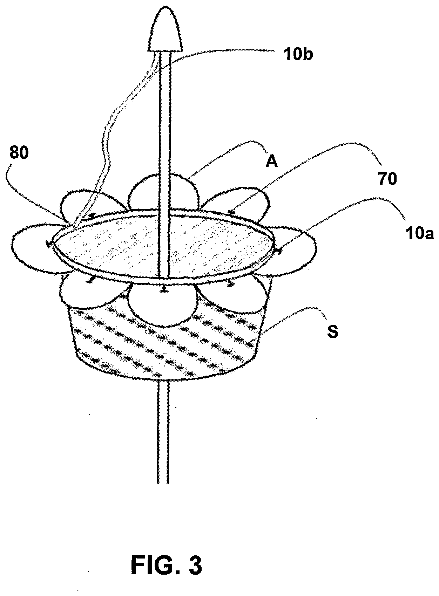

Nach einer weiteren, in Fig. 3 dargestellten Ausführung der Erfindung ist vorgesehen, einen Stent S mit einem ringförmigen Abschnitt 10a auszubilden, der aus wenigstens zwei aufeinanderfolgend angeordneten Abschnitten 20 mit wenigstens zwei Hohlräumen 90, 100/110 gebildet ist, wobei die Hohlräume 90, 100/110 mittels Schleusen 60 abschnittsweise unterteilt sind, und wobei der eine Hohlraum 90 mit wenigstens einer in jedem Abschnitt 20 die Wandung des ringförmigen Abschnitts durchbrechenden Öffnung 70 kommunizierend ausgebildet ist und der andere Hohlraum 100/110 zum Einbringen eines eine Längenänderung wenigstens eines der Abschnitte 20 bewirkenden Elements 40/50 eingerichtet ist.

Durch diese Ausbildung ist es möglich, den Stent S mittels des oben für den Katheter 10a beschriebenen Vorgehensweise an die anatomischen Gegebenheiten eines Gefäßes, beispielsweise das Herz, anzupassen. Dabei weist der Stent S bevorzugt weitere, zusätzliche Verankerungsmittel A auf.

Obwohl vorstehend ausschließlich anhand der Ausbildung des Katheters 10a, des Kathersystems 10 und des Stents S für Anwendungen am menschlichen Herz beschrieben, versteht es sich, dass der erfindungsgemäße Katheter 10a, das Kathetersystem 10 und der Stent S für alle Arten von Gefäßen, wie auch beispielsweise für Ausführgänge von Drüsen oder in Körperhöhlen und anderen unterschiedlich ausgebildeten Körperabschnitten verwendet werden können. Dabei muss der Katheter 10a einen Gewebeabschnitt nicht notwendigerweise umschließen, sondern kann auch zur Erweiterung/Einengung eines Hohlraums innen an der Innenwandung des Hohlraums anliegen.

## Patentansprüche

1. Kathetersystem (10),
mit einem Katheter (10a), mit einem flexiblen, entlang seiner Längserstreckung wenigstens zwei Hohlräume (90, 100/110) ausbildenden Katheterkörper, wobei
der Katheter (10a) ringförmig um eine zu rekonstruierende Struktur legbar und an dieser dauerhaft fixierbar ist, und **gekennzeichnet dadurch dass**
der Katheterkörper wenigstens zwei aufeinanderfolgend angeordnete Abschnitte (20) mit zwischen den Abschnitten (20) angeordneten, die Hohlräume (90, 100/110) abschnittsweise abdichtenden Schleusen (60) aufweist,
wobei
- die einzelnen Abschnitte des Katheters in der Länge veränderbar sind,
- der eine Hohlraum (90) mit wenigstens einer in jedem Abschnitt (20) die Katheterwandung durchbrechenden Öffnung (70) kommunizierend ausgebildet ist zur Fixation des Katheters an dem Gewebe,
- der andere Hohlraum (100/110) zum Einbringen eines eine Längenänderung wenigstens eines der Abschnitte (20) bewirkenden Elements (40/50) eingerichtet ist, und
- wobei der eine Hohlraum (90) zum Anlegen eines auf die Öffnungen (70) wirkenden Unterdrucks eingerichtet ist und/oder die Öffnungen (70) zum Abgeben eines den Katheter (10a) am Gewebe fixierenden Haftmittels eingerichtet sind
und mit einem mit dem Katheter (10a) lösbar verbundenen Katheterträger (10b) mit mit den Hohlräumen (90, 100/110) des Katheters (10a) im verbundenen Zustand kommunizierenden Hohlräumen, die zur beweglichen Aufnahme des eine Längenänderung der Katheterabschnitte (20) bewirkenden Elements (40/50) eingerichtet sind.

2. Kathetersystem nach Anspruch 1, wobei die Abschnitte (20) des Katheters (10a) zu ihrer Längenänderung durch Beaufschlagung der Abschnitte (20) mit einem Unter- oder Überdruck eingerichtet sind.

3. Kathetersystem nach einem der vorhergehenden Ansprüche, wobei die Abschnitte (20) des Katheters (10a) zu ihrer Längenänderung durch Aufschäumen eines in das Lumen eines Abschnitts (20) eingebrachten, aushärtenden Materials eingerichtet sind.

4. Kathetersystem nach einem der vorhergehenden Ansprüche, wobei zur Längenänderung wenigstens eines Abschnitts (20) des Katheters (10a) wenigstens ein Bowdenzug vorgesehen ist.

5. Kathetersystem nach einem der vorhergehenden Ansprüche, wobei die Schleusen (60) des Katheters (10a) unter Abdichtung der von den Abschnitten (20) aufgenommenen Teilabschnitte der Hohlräume (90, 100/110) zur Aufnahme von zur Fixierung des Katheters und/oder zur Längenveränderung der Abschnitte (20) dienenden, fluidführenden Leitungen (30, 40/50) eingerichtet sind.

6. Kathetersystem (10) nach einem der vorhergehenden Ansprüche, wobei der Katheter (10a) und der Katheterträger (10b) eine Kopplungseinrichtung (80) aufweisen, die während des Entkopplungsvorgangs des Katheterträgers (10b) vom Katheter (10a) zum fluiddichten Verschließen der Hohlräume (90, 100/110) des Katheters (10a) eingerichtet ist.

7. Kathetersystem (10) nach einem der vorhergehenden Ansprüche, wobei der Katheter (10a) und der Katheterträger (10b) von einem den Katheter (10a) und den Katheterträger (10b) aufnehmenden Hüllkatheter (5) aufgenommen sind, wobei der Hüllkatheter (5) an seinem freien Ende auslenkbar eingerichtet ist.

8. System mit
- einem Stent (S),
mit einem ringförmigen Abschnitt (10a), welcher um eine zu rekonstruierende Struktur legbar und an dieser dauerhaft fixierbar ist und **gekennzeichnet dadurch, dass** der ringförmige Abschnitt aus wenigstens zwei aufeinanderfolgend angeordneten Abschnitten (20) mit wenigstens zwei Hohlräumen (90, 100/110) gebildet ist, wobei die Hohlräume (90, 100/110) mittels Schleusen (60) abschnittsweise unterteilt sind, und
wobei
- die einzelnen Abschnitte in der Länge veränderbar sind,
- der eine Hohlraum (90) mit wenigstens einer in jedem Abschnitt (20) die Wandung des ringförmigen Abschnitts durchbrechenden Öffnung (70) kommunizierend ausgebildet ist zur Fixation des Stents an dem Gewebe und
- der andere Hohlraum (100/110) zum Einbringen eines eine Längenänderung wenigstens eines der Abschnitte (20) bewirkenden Elements (40/50) eingerichtet ist, wobei der eine Hohlraum (90) zum Anlegen eines auf die Öffnungen (70) wirkenden Unterdrucks eingerichtet ist und/oder die Öffnungen (70) zum Abgeben eines den Abschnitt ' (10a) am Gewebe fixierenden Haftmittels eingerichtet sind, und mit einem mit dem Stent (S) lösbar verbundenen Katheterträger (10b) mit mit den Hohlräumen (90, 100/110) des Stents (S) im verbundenen Zustand kommunizierenden Hohlräumen, die zur beweglichen Aufnahme des eine Längenänderung der Abschnitte (20) bewirkenden Elements (40/50) eingerichtet sind.

9. System nach Anspruch 11, wobei der Stent (S) Verankerungsmittel (A) zum Verankern des Stents (S) im Gewebe aufweist.

## Claims

1. Cather system (10),
with a catheter (10a) that has a flexible catheter body that forms at least two cavities (90, 100/110) along its longitudinal extension, wherein
the catheter (10a) can be placed circularly around and permanently attached to a structure to be reconstructed and is **characterized in that**
the catheter body comprises at least two successively arranged sections (20) with sluices (60) arranged between the sections (20) that seal off the cavities (90, 100/110) section by section,
wherein
- the length of the individual sections of the catheter can be changed,
- one of the cavities (90) is communicatively implemented with at least one opening (70) in each section that penetrates the catheter walling and that serves to attach the catheter to the tissue,
- the other cavity (100/110) is designed for the insertion of at least one element (40/50) that changes the length of at least one of the sections (20), and
- wherein one cavity (90) is designed to apply a vacuum that acts on the openings (70) and/or the openings (70) are designed to emit an adhesive material that fixes the catheter (10a) to the tissue
and with a removably attached catheter carrier (10b) that has cavities which, in the attached state, communicate with the cavities (90 100/110) of the catheter (10a), and are designed for the moveable insertion of the element (40/50) that changes the length of the catheter sections (20).

2. Catheter system in accordance with claim 1,
wherein the sections (20) of the catheter (10a) are designed to be changed in their length by applying a vacuum or an over-pressure to the sections (20).

3. Catheter system in accordance with either of the preceding claims,
wherein the sections (20) of the catheter (10a) are designed to be changed in their length by foaming a hardening material introduced into the lumen of a section (20).

4. Catheter system in accordance with any of the preceding claims,
wherein at least one Bowden wire is provided for changing the length of at least one section (20) of the catheter (10a).

5. Catheter system in accordance with any of the preceding claims,
wherein, while sealing off the subsections of the cavities (90, 100/110) contained in the sections (20), the sluices (60) of the catheter (10a) are designed for the insertion of liquid-conveying conduits (30 40/50) that serve to attach the catheter and/or to change the length of the sections (20).

6. Catheter system in accordance with any of the preceding claims,
wherein the catheter (10a) and the catheter body (10b) comprise a coupling device (80) that is designed to seal off the cavities (90, 100/110) of the catheter (10a) against liquid during the process of decoupling the catheter carrier (10b) from the catheter (10a).

7. Catheter system in accordance with any of the preceding claims,
wherein the catheter (10) and the catheter carrier (10b) are contained in a tunneled catheter (5) that includes the catheter (10) and the catheter carrier (10b), wherein the tunneled catheter (5) is designed to be deflectable at its free end.

8. System with
- a stent (S),
which has a circular section (10a) which can be placed circularly around and permanently attached to a structure to be reconstructed,
**characterized in that** the circular section is formed of at least two successively arranged sections (20) that have at least two cavities (90, 100/110), wherein the cavities (90, 100/110) are divided into sections by means of sluices (60), and wherein
- the length of the individual sections can be changed,
- one of the cavities (90) is communicatively implemented with at least one opening (70) that penetrates the walling of the circular section in every section (20) to fix the stent to the tissue and
- the other cavity (100/110) is designed for the insertion of an element (40/50) for changing the length of at least one of the sections (20),
wherein the cavity (90) is designed to apply a vacuum that acts on the openings (70) and/or the openings (70) are designed to emit an adhesive material that fixes the section (10a) to the tissue,
and with a catheter carrier (10b) removably attached to the stent (S) that has cavities which, in the attached state, communicate with the cavities (90 100/110) of the stent (S), and are designed for the moveable insertion of the element (40/50) that changes the length of the sections (20).

9. System in accordance with claim 8, wherein the stent (S) comprises anchoring means (A) to anchor the stent (S) to the tissue.

## Revendications

1. Système de cathéter (10),
comprenant un cathéter (10a) avec un corps de cathéter flexible formant, selon son extension longitudinale, au moins deux cavités (90, 100/110),
le cathéter (10a) se plaçant selon une forme annulaire autour d'une structure à reconstruire et se fixant de manière permanente à celle-ci,
**caractérisé en ce que**
le corps de cathéter comporte au moins deux segments (20) qui se suivent avec entre les segments (20), les cavités (90,100/110) ayant des sas (60) assurant l'étanchéité par segments,
dans lequel
- les différents segments du cathéter sont de longueur variable,
- la cavité (90) est réalisée avec une ouverture (70) traversant la paroi du cathéter dans chaque segment (20), pour fixer le cathéter au tissu,
- l'autre cavité (100/110) est conçue pour introduire un élément (40/50) réalisant une variation de longueur d'au moins l'un des segments (20), et
- dans lequel
la cavité (90) est conçue pour appliquer une dépression à l'une des ouvertures (70) et/ou les ouvertures (70) sont conçues pour distribuer un agent d'accrochage fixant le cathéter (10a) au tissu, et
un support de cathéter (10b) relié de manière amovible au cathéter (10a), avec les cavités communiquant à l'état relié avec les cavités (90, 100/110) du cathéter (10a), et conçues pour recevoir, de manière mobile, l'élément (40, 50) qui produit la variation de longueur des segments de cathéter (20).

2. Système de cathéter selon la revendication 1,
selon lequel
les segments (20) du cathéter (10a) sont conçus pour faire varier leur longueur en sollicitant les segments (20) avec une pression ou une dépression.

3. Système de cathéter selon l'une des revendications précédentes,
selon lequel
les segments (20) du cathéter (10a) sont conçus pour leur variation de longueur, par l'expansion d'une mousse de matière durcissable introduite dans le lumen d'un segment (20).

4. Système de cathéter selon l'une des revendications précédentes,
selon lequel
au moins un segment (20) du cathéter (10a) comporte au moins un câble Bowden pour la variation de longueur.

5. Système de cathéter selon l'une des revendications précédentes,
selon lequel
en réalisant l'étanchéité des segments partiels des cavités (90, 100/110) pour les segments (20), les sas (60) du cathéter (10a) sont conçus pour recevoir les conduites de fluide (30, 40/50) pour fixer le cathéter et/ou pour la variation de longueur des segments (20).

6. Système de cathéter (10) selon l'une des revendications précédentes,
selon lequel
le cathéter (10a) et le support de cathéter (10b) comportent une installation de couplage (80), conçue pour fermer de manière étanche au fluide, les cavités (90, 100/110) du cathéter (10a) pendant l'opération de découplage du support de cathéter (10b) par rapport au cathéter (10a).

7. Système de cathéter (10) selon l'une des revendications précédentes,
selon lequel
le cathéter (10a) et le support de cathéter (10b) sont dans un cathéter enveloppe (5) recevant le cathéter (10a) et le support de cathéter (10b),
le cathéter enveloppe (5) étant conçu pour pouvoir être dévié à son extrémité libre.

8. Système comprenant :
- un stent (S) avec un segment annulaire (10a) qui peut s'appliquer autour d'une structure à reconstruire et se fixe de manière permanente à celle-ci et,
**caractérisé en ce que**
le segment annulaire est formé d'au moins deux segments (20) successifs, ayant au moins deux cavités (90, 100/110),
- les cavités (90, 100/110) étant subdivisées par segments par des sas (60), et
- les différents segments étant de longueur variable,
- la cavité (90) est conçue avec, dans chaque segment (20), une ouverture (70) traversant la paroi du segment de forme annulaire pour fixer le stent au tissu, et
- l'autre cavité (100/110) est conçue pour introduire un élément (40/50) produisant une variation de longueur d'au moins l'un des segments (20),
* la cavité (90) conçue pour appliquer une dépression agissant sur l'une des ouvertures (70) et/ou les ouvertures (70) étant conçues pour distribuer un agent d'accrochage qui fixe l'un des segments (10a) au tissu, et un support de cathéter (10b) relié de manière amovible au stent (S), avec les cavités communiquant avec les cavités (90,100/110) du stent (S) à l'état relié, ces cavités étant conçues pour recevoir de manière mobile, l'élément (40/50) qui produit une variation de longueur des segments (20).

9. Système selon la revendication 8,
dans lequel
le stent (S) comporte des moyens d'accrochage (A) pour accrocher le stent (S) au tissu.
